**Europäisches Patentamt**

**European Patent Office**

**Office européen des brevets**

(11) Veröffentlichungsnummer : **0 560 088 B1**

(12) # EUROPÄISCHE PATENTSCHRIFT

(45) Veröffentlichungstag der Patentschrift :
**03.08.94 Patentblatt 94/31**

(51) Int. Cl.$^5$ : **A61K 7/135**

(21) Anmeldenummer : **93102442.6**

(22) Anmeldetag : **17.02.93**

(54) **Mittel zum Blondieren von menschlichen Haaren und Verfahren zu dessen Herstellung.**

(30) Priorität : **10.03.92 DE 4207475**

(43) Veröffentlichungstag der Anmeldung :
**15.09.93 Patentblatt 93/37**

(45) Bekanntmachung des Hinweises auf die
Patenterteilung :
**03.08.94 Patentblatt 94/31**

(84) Benannte Vertragsstaaten :
**AT BE CH DE DK ES FR GB IT LI NL**

(56) Entgegenhaltungen :
**EP-A- 0 003 248**
**EP-A- 0 033 687**
**GB-A- 1 083 007**

(73) Patentinhaber : **GOLDWELL**
**AKTIENGESELLSCHAFT**
**Zerninstrasse 10-18**
**D-64297 Darmstadt (DE)**

(72) Erfinder : **Lorenz, Heribert**
**Ober-Ramstädter Strasse 22**
**W-6101 Gross-Bieberau (DE)**
Erfinder : **Kufner, Frank**
**Stockhausenweg 43**
**W-6100 Darmstadt 13 (DE)**

EP 0 560 088 B1

**Beschreibung**

Die vorliegende Erfindung betrifft ein pulverförmiges Mittel zum Blondieren von menschlichen Haaren mit verbesserten Gebrauchseigenschaften und ein Verfahren zu dessen Herstellung.

Herkömmliche Mittel zum Blondieren bzw. Bleichen von menschlichen Haaren bestehen aus mindestens einem festen Peroxid, insbesondere einem Persulfat, und einem pulverförmigen Trägermaterial. Dieses Pulver wird bei Gebrauch mit einer 6- bis 12prozentigen Wasserstoffperoxid-Lösung angerührt und auf das Haar aufgebracht. Beispiele für derartige Zusammensetzungen finden sich in der einschlägigen Fachliteratur, beispielsweise bei K.Schrader, "Grundlagen und Rezepturen der Kosmetika", 2. Auflage (1989, Hüthig Buchverlag), S. 815 bis 823.

Die Gebrauchseigenschaften dieser Blondierpulver sind jedoch bisher nicht befriedigend. Sie stauben nicht nur bei der Anwendung, sondern sind auch bei der Dosierung nicht exakt handhabbar, wodurch das erwünschte Bleichergebnis beeinträchtigt werden kann.

Es wurde nunmehr gefunden, daß ein pulverförmiges Mittel zum Blondieren von menschlichen Haaren, das die erwähnten Nachteile nicht aufweist, erhalten werden kann, wenn das aus mindestens einer festen Perverbindung und mindestens einem pulverförmigen Trägermaterial bestehende Mittel ein Öl und/oder ein flüssiges Wachs enthält. Das so zusammengesetzte Pulver ist nicht nur völlig staubfrei, sondern ist auch gut rieselfähig und gestattet eine exakte Dosierung und läßt sich daher problemlos mit der Wasserstoffperoxid-Lösung vor Anwendung auf dem Haar vermischen. Diese Eigenschaften können gemäß einer bevorzugten Ausführungsform der Erfindung noch dadurch gesteigert werden, wenn man dem Pulver geringe Mengen eines nichtionischen Tensids, beispielsweise eines Fettalkoholethoxylats oder eines Alkylphenolethoxylats, zusetzt.

Aus der DE-OS 2 023 922 sind bereits granulierte Haarbleichmittel bekannt, die aus Persalzen und wasserlöslichen Bindemitteln, insbesondere Polyvinylpyrrolidon, hergestellt worden sind. Diese Granulate vermögen jedoch die oben angesprochenen Probleme deshalb nicht zu lösen, weil sie einerseits relativ hohe Teilchengrößen im Millimeterbereich aufweisen und beim Anrühren mit wäßrigem Wasserstoffperoxid nur schwer in Lösung gehen. Darüber hinaus ist auch die Herstellung dieser Produkte relativ aufwendig, im Gegensatz zu den erfindungsgemäßen Blondierpulvern, die vorzugsweise durch Aufsprühen des Öles bzw. Flüssigwachses auf das die feste Perverbindung und das pulverförmige Trägermaterial enthaltende Gemisch hergestellt werden.

Zur Herstellung der erfindungsgemäßen Blondierpulver sind im Prinzip alle pflanzlichen und tierischen Öle wie auch synthetischen Öle, beispielsweise Siliconöle, geeignet, sofern sie das Lösungs- bzw. Dispergiervermögen der Zusammensetzung mit der wäßrigen Wasserstoffperoxid-Lösung nicht beeinträchtigen. Geeignete Flüssigwachse sind insbesondere solche, die einen Schmelzpunkt von unter 50 °C aufweisen und dadurch auf die pulverförmige Vorproduktmischung aus festem Persulfat und Trägermaterial aufsprühbar sind.

Der Anteil der Öle bzw. Wachse in den erfindungsgemäßen Zusammensetzungen liegt zwischen 2,5 und 25 Gew.-%, insbesondere zwischen 5 und 15 Gew.-%, besonders bei 8 bis 12 Gewichtsprozent, jeweils berechnet auf die Gesamtzusammensetzung des Mittels.

Verbessert werden können die Gebrauchseigenschaften des erfindungsgemäßen Blondierpulvers noch durch die Mitverwendung geringer Mengen nichtionischer Tenside. Als solche sind insbesondere die bekannten $C_{12}$-$C_{18}$-Fettalkoholpolyglycolether, beispielsweise mit 3 bis 15 Ethylenoxid-Einheiten pro Mol, sowie Alkylphenolpolyglycolether, beispielsweise Nonylphenolpolyglycolether mit 4 bis 10 Ethylenoxid-Einheiten pro Mol, geeignet.

Deren Anteil liegt bei 0,1 bis 2,5 Gew.-%, vorzugsweise 0,15 bis 1, insbesondere bei 0,4 bis 0,8 Gew-%, berechnet auf die Gesamtzusammensetzung des erfindungsgemäßen Mittels.

Das erfindungsgemäße Mittel zum Blondieren bzw. Bleichen von menschlichen Haaren enthält die in solchen Mitteln bekannten und üblichen Bestandteile, es wird hierzu, zur Vermeidung von Wiederholungen, auf Schrader, l.c., verwiesen.

Geeignete Peroxide sind insbesondere Alkalipersulfate wie Kalium- und Ammoniumpersulfat, Magnesiumperoxid, Harnstoffperoxid, Melaminperoxid sowie Gemische derselben.

Die Herstellung des erfindungsgemäßen Blondierungsmittels erfolgt gemäß einer bevorzugten Ausführungsform der Erfindung durch Aufsprühen des Öls oder Flüssigwachses auf die restlichen Pulverbestandteile. Dies geschieht vorzugsweise bei Raumtemperatur, d. h., diese Pulverbestandteile sollen nach Möglichkeit nicht über 30 °C erhitzt werden.

Eine andere Möglichkeit der Zumischung besteht darin, die Pulverbestandteile mit dem Öl bzw. Flüssigwachs in einer Mühle, beispielsweise einer Kugelmühle, zu vermahlen; für die technische Herstellung des Produktes wird jedoch dem obengenannten Sprühverfahren der Vorzug gegeben.

Die Teilchengrößen der erfindungsgemäßen Blondiermittel liegen in der Regel unterhalb 500 μm, vor-

zugsweise unterhalb 400 μm, was eine ausgezeichnete Verarbeitbarkeit, d. h. Mischbarkeit mit der wäßrigen Wasserstoffperoxid-Lösung vor Applikation auf das menschliche Haar, gewährleistet.

Die Anwendung des Mittels geschieht in an sich bekannter und üblicher Weise: Durch Vermischen des pulverförmigen Blondiermittels mit einer 6- bis 12prozentigen Wasserstoffperoxid-Lösung, wobei etwa 1 Teil des Pulvers mit etwa 1,5 Teilen der Peroxid-Lösung, vorzugsweise 9%iger $H_2O_2$-Lösung, homogen vermischt und anschließend etwa 25 bis etwa 45 Minuten auf das Haar zur Einwirkung gebracht wird.

Im folgenden werden einige Beispiele zur Realisierung der Erfindung gegeben.

Beispiel 1

| | |
|---|---|
| Siliciumdioxid (Diatomeenerde) | 3,20 (Gew.-%) |
| Siliciumdioxid (pyrogenes $SiO_2$) | 5,30 |
| Natriumcarboxymethylcellulose | 3,50 |
| Harnstoff | 2,00 |
| Natriumlauroylsarkosinat | 0,80 |
| Natriumstearat | 1,20 |
| Natriumcarbonat | 1,00 |
| Natriummetasilikat | 6,00 |
| Stärkepulver | 3,50 |
| Kaliumpersulfat | 58,00 |
| Magnesiumperoxid | 4,00 |
| Paraffinöl (Paraffinum perliquidum, DAB 9) | 11,50 |

Es wird ein staubfreies Pulver erhalten, das mit einer bekannten 9%igen $H_2O_2$-Lösung im Gewichtsverhältnis 1 : 1,5 gut angerührt werden kann.
99 % der Teilchen hatten einen Durchmesser von < 400 μm.
Die Herstellung des Pulvers erfolgte durch Aufsprühen des Paraffinöls auf die Pulvergrundmasse bei etwa 20 °C im Wirbelsprühverfahren.

Beispiel 2

Die Zusammensetzung nach Beispiel 1 wurde dahingehend verändert, daß zusätzlich, unter entsprechender Verringerung des Gehaltes an Paraffinöl, 0,15 Gew.-% eines $C_{12}$-$C_{14}$-Fettalkoholethoxylats ($\approx$ 6 EO-Einheiten/Mol) zugesetzt wurden.
Es wurde ein fließfähiges staubfreies Produkt erhalten, das mit 9%iger $H_2O_2$-Lösung ausgezeichnet anrührbar war.

Beispiel 3

Die Zusammensetzung nach Beispiel 1 wurde dahingehend verändert, daß zusätzlich, unter entsprechender Verringerung des Gehaltes an Paraffinöl, 0,50 Gew.-% eines Nonylphenolethoxylats ($\approx$4 EO-Einheiten/Mol) zugesetzt wurden.
Das erhaltene fließ- bzw. rieselfähige Blondierpulver staubt nicht und ist mit 9%iger $H_2O_2$-Lösung ausgezeichnet anrührbar.

**Patentansprüche**

1. Pulverförmiges Mittel zum Blondieren von menschlichen Haaren, enthaltend mindestens eine feste Perverbindung und mindestens ein pulverförmiges Trägermaterial, dadurch gekennzeichnet, daß es 2,5 bis 25 Gew.-%, berechnet auf die Gesamtzusammensetzung, eines Öls oder flüssigen Wachses enthält.

2. Mittel nach Anspruch 1, dadurch gekennzeichnet, daß es 5 bis 15 Gew.-% eines Öles und/oder flüssigen Wachses enthält.

3. Mittel nach einem der Ansprüche 1 oder 2, dadurch gekennzeichnet, daß es Paraffinöl enthält.

4. Mittel nach einem der vorhergehenden Ansprüche, dadurch gekennzeichnet, daß es 0,1 bis 1 Gew.-% eines nichtionischen Tensids enthält.

5. Mittel nach Anspruch 4, dadurch gekennzeichnet, daß es als nichtionisches Tensid einen Fettalkoholpolyglycolether und/oder einen Alkylphenolpolyglycolether enthält.

6. Verfahren zur Herstellung eines pulverförmigen Mittels zum Blondieren von menschlichen Haaren nach einem der Ansprüche 1 bis 5, dadurch gekennzeichnet, daß auf ein mindestens eine feste Perverbindung und mindestens einen pulverförmigen Träger enthaltendes Pulver ein Öl und/oder ein flüssiges Wachs aufgesprüht wird.

**Revendications**

1. Composition pulvérulente pour la décoloration des cheveux humains, comprenant au moins un composant peroxyde solide et au moins un composant de support poudreux, caractérisée en ce qu'elle contient 2,5 à 25 % en poids, calculé à la composition totale, d'une huile ou d'une cire liquide.

2. Composition selon la revendication 1, caractérisée en ce qu'elle contient 5 à 15 % en poids, calculé à la composition totale, d'une huile et (ou) d'une cire liquide.

3. Composition selon la revendication 1 ou 2, caractérisée en ce qu'elle contient d'une huile de paraffine.

4. Composition selon l'une des revendications précédentes, caractérisée en ce qu'elle contient 0,1 à 1 % en poids d'un agent tensio-actif non-ionique.

5. Composition selon la revendication 4, caractérisée en ce qu'elle contient un alcool du gras polyoxyéthyléné et (ou) un alkylphénol polyoxyéthyléné comme agent tensio-actif non-ionique.

6. Procédé pour la préparation d'une composition pour la décoloration des cheveux humains selon une des revendications 1 à 5, caractérisé en ce qu'une huile et (ou) une cire liquide est vaporisée sur un mélange comprenant au moins un composant peroxyde solide et au moins un composant de support poudreux.

**Claims**

1. Pulverulent composition for bleaching of human hair, comprising at least one solid peroxide compound and at least one powdery carrier material, characterized in that it contains, calculated to the total composition, 2.5 to 25% by weight of an oil or liquid wax.

2. Composition according to claim 1, characterized in that it comprises 5 to 15% by weight of an oil and (or) a liquid wax.

3. Composition according to claim 1 or 2 characterized in that it comprises paraffin oil.

4. Composition according to one of the preceding claims, characterized in that it contains 0.1 to 1% by weight of a nonionic surfactant.

5. Composition according to claim 4, characterized in that it contains as a non-ionic surfactant a fatty alcohol polyglycol ether and (or) an alkylphenol polyglycol ether.

6. Process for the preparation of a pulverulent composition for bleaching of human hair according to one of claims 1 to 5, characterized in that an oil and (or) a liquid wax is sprayed onto a mixture comprising at least one solid peroxide compound and at least one pulverulent carrier material.